# NEUE EUROPÄISCHE PATENTSCHRIFT

(11) **EP 1 317 188 B2**
(45) Veröffentlichungstag und Bekanntmachung des Hinweises auf die Entscheidung über den Einspruch: **11.06.2008**
(45) Hinweis auf die Patenterteilung: 01.12.2004
(21) Anmeldenummer: 01984622.9
(22) Anmeldetag: 14.09.2001
(51) Int. Cl.: A23K 1/16, A61P 1/00, A61K 31/47

(54) **VERWENDUNG VON BENZOPHENANTHRIDINALKALOIDEN ALS FUTTERZUSATZMITTEL**
USE OF BENZOPHENANTHRIDINE ALKALOIDS AS FEED ADDITIVES
UTILISATION DE BENZOPHENANTRIDINE ALKALOIDES COMME ADDITIFS ALIMENTAIRES POUR ANIMAUX

(30) Priorität: 15.09.2000 AT 15790000
(43) Veröffentlichungstag der Anmeldung: 11.06.2003
(73) Patentinhaber: Roth, Herrmann, 65343 Eltville (DE)
(72) Erfinder: Roth, Herrmann, 65343 Eltville (DE)
(74) Vertreter: Puchberger, Peter
(86) Internationale Anmeldenummer: PCT/IB2001/002487
(87) Internationale Veröffentlichungsnummer: WO 2002/021932

(56) Entgegenhaltungen:
- WO-A-02/21932
- WO-A-93/16602
- US-A- 5 175 000
- J. DRSATA ET AL.: "Sanguinarine and chelerythrine as inhibitors of aromatic amino acid decarboxylase" JOURNAL OF ENZYME INHIBITION, Bd. 10, Nr. 4, 1996, Seiten 231-237, XP001064019 NEW YORK, NY, US
- S. MELLOR: "Natural appetisers from plants" FEED MIX, Bd. 9, Nr. 1, 2001, Seiten 29-31, XP001064055 DOETINCHEM, NL ISSN: 0928-124X
- N.P. TUONG: "Note sur l'utilisation du tannate de berbérine (Bertamix) comme activateur de croissance dans l'élevage des porcs au Vietnam" REVUE D'ELEVAGE ED DE MEDECINE VETERINAIRE DES PAYS TROPICAUX, Bd. 44, Nr. 3, 1991, Seiten 361-362, XP001065211
- PATENT ABSTRACTS OF JAPAN vol. 013, no. 447 (C-642), 6. Oktober 1989 (1989-10-06) & JP 01 172339 A (NISSHIN FLOUR MILLING CO LTD), 7. Juli 1989 (1989-07-07)
- P. PRZYBILLA ET AL, 1998, DGS Magazin, Woche 40, pp. 52-57
- B.D. Krane et al, 1984, Journal of natural products, vol. 47, n°1, pp. 1-43
- Tabel (Table), 2005, published by the CVB Centraal Veevoederbureau (the Central Animal Feed Bureau)
- R.I. Mackie et al, 1998, J. Anim. Sci., vol. 76, pp. 1331-1342
- G.L. Cromwell et al, J. Anim. Sci. 1991, vol. 76, pp. 4898-4906
- A. Sundrum et al, 2000, J. Anim. Sci., vol. 79, pp. 1199-1205

## Beschreibung

Diese Erfindung betrifft die Verwendung eines oder mehrerer Benzophenanthridinalkaloide oder ihrer Derivate oder synthetischen Analoge als Futteradditiv zum Hemmen oder Unterbinden des enzymatischen Abbaus der essentiellen aromatischen Aminosäuren in einem Futtermittel sowie die Verwendung von Benzophenanthridinalkaloiden zur Herstellung eines Heilmittels.

Aromatische Aminosäuren wie z.B. Tryptophan und Alanin sind essentielle Nährstoffe, die der Körper nicht selbst herstellen kann, ausgenommen Wiederkäuer. Sie sind ein wesentlicher Bestandteil der Rezeptur von Nahrung und Tierfutter. Im Tierfutter dienen sie dazu, wie andere Aminosäuren auch, in einem bestimmten Verhältnis zum Gehalt anderer essentieller Aminosäuren die erforderliche Balance zwischen den einzelnen essentiellen und nicht essentiellen Aminosäuren zu gewähren. Ihr Einsatz verursacht Kosten.

Im Darm von Säugetleren kommen weit verbreitet Mikroorganismen vor, die in der Lage sind, mit Hilfe ihrer Enzyme. z.B. der Aromatischen Aminosäuren-Decarboxilase. diese Aminosäuren abzubauen. Als Resultat ist die anfänglich bei der Futtermittelbereitung zugrunde gelegte Balance zwischen den einzelnen Aminosäuren nicht mehr gegeben. Die Fleischqualität von Tieren wird herabgesetzt.

Als Zwischenprodukt des enzymatischen Abbaus aromatischer Aminosäuren entstehen Indol und Skatol, die unerwünschte Eigenschaften und Wirkungen hervorrufen. Indol und Skatol sind toxische Stoffe des Tryptophanbbbaus. Sie belasten u.a. die Leber, da der Körper diese Substanzen entgiften muss, um nicht daran zu erkranken. Indol und Skatol führen zu chronischen subklinischen Entzündungen der Schleimhautgewebe betroffener Säugetiere und Menschen. Indol und Skatol, produziert durch Mikroorganismen der Darmschleimhaut, erleichtern pathogenen Parasiten (Darmwürmer, Clostridien, Salmonellen, Kokzidien) den Eintritt (Invesion) in die oberen Zellen der Darmschlelmhaut bzw. vermindern deren Abwehrbereitschaft.

Darüber hinaus kann ein Rückstand an Indol und Skatol in der Nahrung auch den Konsumenten gesundheitlich oder im Wohlbefinden ungünstig beeinflussen. Eine ungünstige Relation zwischen aromatischen Aminosäuren (in Relation zu niedrige Menge verfügbar) und den weiteren essentiellen Aminosäuren führt zu einer Imballance, die einen verminderten Proteineinsatz und einen verminderten Milcheiweißgehalt als Folge hat. Nahrungsqualität wird auch nach Proteingehalt bewertet. Ein wegen eines Mangels an aromatischen Aminosäuren verminderter Proteingehalt (Milcheiweiß, Magerfleisch) führt zu verminderter Qualität und Preisabschlägen.

Die Aromatische Aminosäuren-Decarboxilase wird im Zusammenhang mit der Parkinsonschen Erkrankung beim Menschen als eine mögliche Ursache gesehen, die mitteibar zur Erkrankung führt. Eine Vielzahl an Mikroorganismen, die im Darm und Darmschleimhaut von Tieren leben, produzieren Protein- und Aminosäuren abbauende Enzyme. Kritisch wird die Situation dann, wenn essentielle Nährstoffe von diesem Abbauprozess betroffen sind und des weiteren toxische Abbauprodukte entstehen, die den Wirt belasten oder zu Rückständen in Nahrungsmittein führen. Die Kosten der Futteroptimierung werden insofern erhöht, als der bisher unvermeidliche Verlust an aromatischen Aminosäuren bereits durch eine höhere Zumischung im Futtermittel Berücksichtigung fand. Als Konsequenz der Entstehung und unbeeinflussten Aktivität der Aromatischen Aminosäuren- Decarboxilase sind die Kosten der Ernährung und der Gesunderhaltung von Nutzund Hobbytieren erhöht.

Das vorliegend beschriebene Futtermittel ist in erster Linie dadurch gekennzeichnet, dass es zur Verringerung des enzymatischen Abbaus der essentiellen aromatischen Aminosäuren ein oder mehrere Benzophenanthridinalkaloide oder deren Derivate oder synthetische Analoge in wirksamer Menge verwendet.

Aus WO-A-9 316 602 (Neufeld) ist die Verwendung von Sanguinarin und anderen Benzophenanthridinalkaloiden als Leistungsförderer bei Nutztieren, die Verwendung dieser Alkaloide zur Behandlung bzw. Prophylaxe gegen Salmonellenbefall, gegen Kokzidien und andere bakterielle Erreger und zur Futtermittelkonservierung bekannt geworden.. Der Einsatz dieser Alkaloide zum Hemmen oder Unterbinden des enzymatischen Abbaus der AAD (Aromatische Aminosäure-Decarboxylase) wird darin nicht erwähnt.

Drata et al., J. Enzyme Inhibition, 1996, Vol. 10, p. 231-237, berichten über die Wirkung von Sanguinarin als Inhibitor der AAD, die eine wichtige Rolle bei der Biosynthese von Dopamin, Noradrenalin, Adrenalin und Serotonin spielt. Da Dopamin im Zusammenhang mit der Parkinsonschen Krankheit von Bedeutung ist, Noradrenalin ein potenter Vasokonstriktor ist und Serotonin zu den Entzündungsmediatoren zählt, ist die Hemmung der AAD Gegenstand der Studie. Aufgrund der dort gewonnenen Erkenntnisse ist die Verwendung von Benzophenanthridinalkaloiden zur Verbesserung der Proteinbilanz bei Nutztieren oder ihre Verwendung als Heilmittel im veterinärmedizinischen Bereich nicht nahe gelegt.

Erfindungsgemäß werden ein oder mehrere Benzophenanthridinalkaloide oder ihre Derivate oder synthetischen Analoge als Futteradditiv in wirksamer Menge zum Hemmen oder Unterbinden des enzymatischen Abbaus der essentiellen aromatischen Aminosäuren in einem Futtermittel oder Futterzusatzmittel verwendet. Gemäß einem weiteren Merkmal der Erfindung können die Benzophenanthridinalkaloide in Form von Pflanzenmaterial der Papaveraceae oder als Extrakt davon oder in Form der isolierten Alkaloide oder Alkaloidgemische oder synthetische Analoge zum Einsatz kommen.

Gemäß einem weiteren Merkmal der Erfindung werden ein oder mehrere Benzophenanthridinalkaloide oder ihre Derivate oder synthetischen Analoge oder Benzophenanthridinalkaloid(e) enthaltende Pflanzenbestandteile der Papaveraceae zur Herstellung von Fultermitteln oder Futterzusatzmitteln zur Verbesserung der Proteinbilanz bei Nutztieren durch Hemmen oder Unterbinden des enzymatischen Abbaus von aromatischen Aminosäuren im Verdauungstrakt verwendet.

Ein weiterer Gegenstand der Erfindung ist die Verwendung von Benzophenanthridinalkaloiden oder ihrer Derivate oder synthetischen Analoge oder von Benzophenanthridinalkaloid(e) enthaltenden Pflanzenbestandteilen der Papaveraceae zur Herstellung eines Heilmittels gegen Darmentzündungen, insbesondere solche, die durch Abbauprodukte des Aminosäurestoffwechsels, wie Indol und Skatol, hervorgerufen sind.

Chelerythrin und Sanguinarin sowie andere analoge Benzophenanthridinalkaloide haben sich als potente Inhibitoren der Aromatischen Aminosäuren-Decarboxilase erwiesen. Es konnte gezeigt werden, dass Sanguinarin und Chelerythrin die Aktivität der Aromatischen Aminosäuren-Decarboxilase zu 99 % bzw. 92 % blockieren. Diese Blockade der Aromatischen Aminosäuren-Decarboxilase ist irreversibel und daher von besonderem Interesse, da sie solange anhält, wie sich das Enzym-Molekül im Wirkungsbereich der Benzophenanthridinalkaloide befindet. Sanguinarin und Chelerythrin, Vertreter der Benzophenanthridinalkaloide, haben endständig eine aromatische Struktur, die derjenigen der aromatischen Aminosäuren ähnlich ist. Das Enzym "Aromatische Aminosäuren-Decarboxilase" verwechseit vermutlich die endständige Struktur der Benzophenanthridinalkaloide mit der Ringstruktur der aromatischen Aminosäuren und baut diese anstelle der Aminosäure in sein aktives Zentrum ein. Da Benzophenanthridinalkaloide kein abspaltbares Kohlenstoffskelett besitzen, wie dies bei den Aminosäuren der Fall ist, gibt es kein Endprodukt der enzymatischen Reaktion, wodurch diese nicht beendet werden kann. Das aktive Zentrum der Aromatischen Aminosäuren-Decarboxilase ist irreversibel blockiert. Die Erfindung zeigt, dass es möglich ist, die Aktivität und damit die Soffwechselprodukte der Aromatischen Aminosäuren-Decarboxilase durch Zugabe einer den aromatischen Strukturen der aromatischen Aminosäuren ähnlichen Struktur irreversibel zu blockieren und dies für die Tiernahrung und als Heilmittel vorteilhaft auszunützen.

Die Erfindung führt zu einem reduzierten Abbau und Verlust essentieller aromatischer Aminosäuren und dadurch zu einer besseren Versorgung des Tieres mit diesen Nährstoffen. Versuche zeigen, dass sich dadurch der Magerfleischgehalt und der Gehalt an Milcheiweiß deutlich, zum Teil signifikant verbessern lassen. Weitere Versuche zeigen, dass durch die sohin verbesserte Balance innerhalb des Aminosäutemusters die Proteinbilanz bei Schweinen in der Endmast um bis zu 10 % verbessert ist.

Die Erfindung führt zu einer verbesserten Tiergesundheit. Versuche zeigen, dass der Einsatz der Benzophenanthridinalkaloide zu einer deutlich verminderten Entzündungsfrequenz im Darm von Tieren resultiert.

Das Futtermittel kann alle herkömlichen Futterstoffe enthalten, wie sie in der Tierhaltung üblich sind. Darunter fallen die stärkehaltigen Bestandteile wie Getreide, Getreideprodukte und Mais. Die weiters enthaltenen Proteine können natürlichen Ursprungs sein oder auch als Präparate hinzugesetzt werden, was insbesondere für die essentiellen Aminosäuren gilt. Welters können mineralische Zuschlagstoffe wie Spurenelemente, Phosphate, Salze und Kalk vorhanden sein. Weitere Zusatzstoffe wie Enzyme, Vitamine oder medikamentös wirkende Stoffe können ebenfalls zugesetzt werden.

Die im Futtermittel enthaltenen aromatischen Aminosäuren können entweder oder zum Teil natürlichen Ursprungs oder künstliche Zusätze sein. Wesentliche aromatische Aminosäuren sind z.B. Thryptophan und Phenylalanin.

Die Benzophenanthridinalkaloide kommen in der Natur beispielsweise in den Rhizomen der Kanadischen Blutwurzel Sanguinaria Canadensis oder auch in Chelidonium majus oder Macleaya cordata vor. Diese Pflanzen zählen zu den Papaveraceae. Die Rhizome oder das Blattmaterial oder das gesamte Pflanzenmaterial können gesammelt und getrocknet werden. Bezogen auf die Trockensubstanz beträgt der Gehalt an Benzophenanthridinalkaloiden üblicherweise ca. 4 Gew.-%. Um Emteschwankungen auszugleichen, kann es vorteilhaft sein, den Gehalt des Hauptalkaloides Sanguinarin auf einen Wert von z.B. 1,5 Gew.-% durch Hinzugabe von Füllstoffen einzustellen. Da etwa die Hälfte des natürlichen Alkaloidgehaltes auf das Sanguinarin entfällt, liegt somit der Gesamtalkaloidgehalt bei etwa 3 Gew.-%.

Weitere Alkaloide dieser Gruppe sind Chelerythrin, Chelirubin, Sanguirubin, Chelilutin und Sanguilutin.

Im Rahmen der Erfindung kann es auch vorteilhaft sein, Extrakte des Pflanzenmateriales oder die isolierten Alkaloide und deren Derivate oder deren synthetische Analoge zu verabreichen.

Die im Futter enthaltene Alkaloidmenge ist nach unten lediglich durch die Wirksamkeit begrenzt. Die Gesamtalkaloidmenge pro Tonne Futtermittel ist bevorzugt größer 1 mg. Bezogen auf das standardisierte getrocknete Papaveraceae-Wurzelmaterial entspricht dies etwa 0,067 g je Tonne Futtermittel.

In der bisher genauer untersuchten Futtermittelverabreichung wurden Mengen zwischen 5 g und 150 g getrockneter Papaveraceae je t Futter, standardisiert auf 1,5 % Alkaloid Sanguinarin, entsprechend ca. 3 % Gesamtalkaloid, verabreicht.

Die Verwendung von Benzophenanthridinalkaloiden erfolgt auch in Futterzusatzmitteln wie z.B. Vormischungen (Premixe) oder in Mineralfutter oder Ergänzungsfuttermittel zur Zubereitung von Futtermittel, wobei das Zusatzmittel neben den üblichen Futterzusatzstoffen Benzophenanthridinalkaloide oder deren Derivate und Analoge oder diese Alkaloide enthaltende Pflanzenbestandteile der Papaveraceae enthält.

Weiters betrifft die Erfindung die Verwendung von Benzophenanthridinalkaloiden oder deren Derivaten und Analogen oder von diesen enthaltenden Pflanzenbestandteilen der Papaveraceae zur Hemmung oder Unterbindung des enzymatischen Abbaus von aromatischen Aminosäuren, insbesondere im Verdauungstrakt von Tieren, insbesondere in Kombination mit einem der vorbeschriebenen Futter- oder Futterzusatzmittel.

Wenn im Tierfutter genügend essentielle aromatische Aminosäuren enthalten sind, kann die Zugabe der Alkaloide die zusätzliche Verabreichung der teuren aromatischen Aminosäuren ersparen. Falls aromatische Aminosäuren zugesetzt werden müssen, wie z.B. Thryptophan und Phenylalanin, dann kann die zugesetzte Menge durch das Alkaloid herabgesetzt werden, wodurch Kosten eingespart werden. Als Richtwert, bezogen auf heutige Marktverhältnisse, kann man eine Ersparnis von etwa 10 EURO Futtermittelpreis annehmen, wenn erfindungsgemäß Alkaloidpräparate im Wert von 1 EURO zum Futtermittel hinzugesetzt werden.

Die Benzophenanthridinalkaloide können gemäß Erfindung auch vorteilhaft als Heilmittel gegen Darmentzündung verwendet werden, insbesondere solche, die durch Abbauprodukte des Aminosäurestoffwechsels wie Indol oder Skatol hervorgerufen sind. Gekennzeichnet ist das Heilmittel durch einen Gehalt einer wirksamen Menge an Benzophenanthridinalkaloiden oder deren Derivaten oder synthetischen Analogen oder diese enthaltende Pflanzenbestandteile der Papaveraceae.

Der Einsatz der Heilmittel erfolgt bevorzugt gemeinsam mit Futtermittel oder als Zusatz zum Trinkwasser. Auch die wirksamen Mengen der Alkaloide liegen in den gleichen Bereichen.

Nachfolgend werden einige Ausführungsbeispiele beschrieben.

### 1. Beispiel einer herkömmlichen Füttermittelrezeptur für Hühner und Schweine

| (in Gewichtsprozent) | |
|---|---|
| Weizen: | 42 % |
| Mais: | 20 % |
| Sonnenblumen: | 3,6 % |
| Soyaschrot I | 4 % |
| Soyaschrot II | 16,6 % |
| Rapsschrot | 4 % |
| Futteröl | 6,5 % |
| Methionin | 0,26 % |
| Lysin | 0,48 % |
| Mono Calcium Phosphat | 0,85 % |
| Kalk | 0,5 % |
| Salz | 0,025 % |
| Vormischung | 1,0 % |
| Enzyme | 0,2 % |

| Aminosäuren: | |
|---|---|
| Protein: | 19,2 % |
| Lysin | 0,824 % |
| Methionin | 0,276 % |
| Meth + Cystin | 0,568 % |
| Threonin: | 0,8 % |
| Thryptophan | 0,24 % |

### 2. Beispiel gemäß Erfindung:

Das Futtermittel für Schweine entspricht Beispiel 1. Die angegebenen Mengen zugesetzte Papaveraceae sind in g getrocknetes Wurzelmaterial mit 1,5 Gew.-% Alkaloid Sanguinarin pro Tonne Futtermittel. Tierrasse: Great Yorkshire + Kreuzungssauen, pro Gruppe 23-24 Tiere.

| Gruppen untersuchte Tiere: | | a | b | c |
|---|---|---|---|---|
| Papaveraceae g/t | Starter/Vormast 22 kg - 48 kg | 0 | 0 | 15 |
| | Endmast 48 kg - 109 kg | 0 | 30 | 30 |

### Resultat:

### Klassifizierung und Schlachtkörperwert

(AA besser als A besser als B):

| | O-Kontrolle | Papaveraceae-Dosierungen g/t Vormast/Endmast | |
|---|---|---|---|
| | (0/0) | (0/30) | (15/30) |
| Magerfleisch % | 54.5 | 55.1 | 55.0 NS |
| Rückenspeck mm | 17.8 | 17.7 | 17.6 NS |
| Muskelfläche | 51.9 | 55.6 | 54.2 (p <0.05) |
| Klasse AA + A % | 77 % | 96 % | 92 % |
| Klasse B | 23 % | 4 % | 8 % |

Somit ist gemäß der Erfindung der Anteil der Klasse AA+A signifikant erhöht.

### 3. Beispiel:

| | | |
|---|---|---|
| Gruppen: | 0-Kontrolle | 30g Papaveraceae/t Futtermittel |
| | 144 Ferkel am Start, 72 je Gruppe, verteilt auf | |
| | 8 Wiederholungen je Gruppe. | |
| | Restriktive Fütterung. | |

### Vormast-Endmast 31-115 kg:

| | 0-Kontrolle | Papaveraceae 30g/t |
|---|---|---|
| Magerfleisch (FOM)% | 54.9 | 55.2 |
| Muskelfläche cm² | 51.7 | 52.0 |
| Rückenspeck cm | 20.6 | 20.1 |

Wirtschaftlichkeit: Basis 1.022 Euro/kg Schlachtgewicht

| | |
|---|---|
| Schlachtkörperwert Euro | +1.53 |
| Papaveraceae Vorteil Euro | +2.71 |

### Kommentar:

In einem System mit restriktiver Fütterung erhöht Papaveraceae den Schlachtkörperwert, indem der Anteil an Magerfleisch und Muskelfläche steigt und zugleich der Rückenspeckwert sinkt. Dadurch erhöht Papaveraceae die Fleischqualität und die Rentabilität um ca. 1,53 Euro je Tier.

### 4. Beispiel:

Geflügel-Futter:
5 Gruppen zu je 28.000 Geflügel
Kokzidiostatikum Salinomycin

| | Kontrolle | Papaveraceae |
|---|---|---|
| | 15 ppm Virginiamycin (Antibiotica) | 50 g/t |
| Parasitensporen in 1000/g Kot (Coccidien-oocysten) | 5 - 40 | 1 - 6 |
| Lesionindex 0, * bis *** | 0 bis ** | 0 bis * |

Der Versuch zeigte eine deutlich günstigere Bewertung der Darm-Entzündungen und deutlich reduzierte Ausscheidung an Darmparasiten.

## Patentansprüche

1. Verwendung eines oder mehrerer Benzophenanthridinalkaloide oder ihrer Derivate oder synthetischen Analoge als Futteradditiv in wirksamer Menge zum Hemmen oder Unterbinden des enzymatischen Abbaus der essentiellen aromatischen Aminosäuren in einem Futtermittel oder Futterzusatzmittel.

2. Verwendung nach Anspruch 1, **dadurch gekennzeichnet, dass** die Benzophenanthridinalkaloide in Form von Pflanzenmaterial der Papaveraceae oder als Extrakt davon oder in Form der isolierten Alkaloide oder Alkaloidgemische oder synthetische Analoge zum Einsatz kommen.

3. Verwendung nach Anspruch 1 oder 2, **dadurch gekennzeichnet, dass** das Futtermittel essentielle aromatische Aminosäuren und Benzophenanthridinalkaloide enthält.

4. Verwendung nach einem der Ansprüche 1 bis 3, **dadurch gekennzeichnet, dass** die essentiellen aromatischen Aminosäuren dem Futter zusätzlich zugesetzt werden.

5. Verwendung nach einem der vorhergehenden Ansprüche, **dadurch gekennzeichnet, dass** die Alkaloidmenge pro Tonne Futtermittel größer 1 mg ist.

6. Verwendung von Benzophenanthridinalkaloiden oder ihrer Derivate oder synthetischen Analoge oder von Benzophenanthridinalkaloid(e) enthaltenden Pflanzenbestandteilen der Papaveraceae zur Herstellung von Futtermitteln oder Futterzusatzmitteln zur Verbesserung der Proteinbilanz bei Nutztieren durch Hemmen oder Unterbinden des enzymatischen Abbaus von aromatischen Aminosäuren im Verdauungstrakt.

7. Verwendung von Benzophenanthridinalkaloiden oder ihrer Derivate oder synthetischen Analoge oder von Benzophenanthridinalkaloid(e) enthaltenden Pflanzenbestandteilen der Papaveraceae zur Herstellung eines Heilmittels gegen Darmentzündungen, die durch Abbauprodukte des Aminosäurestoffwechsels, wie Indol und Skatol, hervorgerufen sind.

## Claims

1. Use of one or more benzophenanthridine alkaloids or derivatives or synthetic analogues thereof as a feed additive in an effective amount for inhibiting or suppressing the enzymatic decomposition of the essential aromatic amino acids in a feed or feed additive.

2. Use according to claim 1, **characterised in that** the benzophenanthridine alkaloids are used in the form of plant material of the Papaveraceae or an extract thereof or in the form of the isolated alkaloids or alkaloid mixtures or synthetic analogues.

3. Use according to claim 1 or 2, **characterised in that** the feed comprises essential aromatic amino acids and benzophenanthridine alkaloids.

4. Use according to any one of claims 1 to 3, **characterised in that** the essential aromatic amino acids are additionally added to the feed.

5. Use according to any one of the preceding claims, **characterised in that** the amount of alkaloid per tonne of feed is greater than 1 mg.

6. Use of benzophenanthridine alkaloids or derivatives or synthetic analogues thereof or of plant parts of the Papaveraceae containing benzophenanthridine alkaloid(s) in the production of feeds or feed additives for improving the protein balance in useful animals by inhibiting or suppressing the enzymatic decomposition of aromatic amino acids in the digestive tract.

7. Use of benzophenanthridine alkaloids or derivatives or synthetic analogues thereof or of plant parts of the Papaveraceae containing benzophenanthridine alkaloid(s) in the production of a medicament against intestinal inflammations,
caused by decomposition products of amino acid metabolism, such as indole and skatole.

## Revendications

1. Utilisation d'un ou de plusieurs alcaloïdes de benzophénanthridine ou leurs dérivés ou leurs analogues synthétiques en tant qu'additifs alimentaires dans une quantité efficace pour inhiber ou bloquer la décomposition enzymatique des acides aminés aromatiques essentiels dans un produit alimentaire ou additif alimentaire.

2. Utilisation selon la revendication 1, **caractérisée en ce que** l'alcaloïde de benzophénanthridine est mis en oeuvre sous la forme de matière végétale provenant des papaveraceae ou en tant qu'extrait de celle-ci ou sous la forme de l'alcaloïde isolé ou mélanges d'alcaloïdes ou d'analogues de synthèse.

3. Utilisation selon la revendication 1 ou 2, **caractérisée en ce que** le produit alimentaire contient des acides aminés aromatiques essentiels et de l'alcaloïde de benzophénanthridine.

4. Utilisation selon l'une des revendications 1 à 3, **caractérisée en ce que** les acides aminés aromatiques essentiels sont ajoutés en tant qu'additifs à l'aliment.

5. Utilisation selon l'une des revendications précédentes, **caractérisée en ce que** la quantité d'alcaloïde par tonne de produit alimentaire est supérieure à 1 mg.

6. Utilisation d'alcaloïdes de benzophénanthridine ou de leurs dérivés ou de leurs analogues synthétiques ou de composants végétaux des papaveraceae contenant un ou des alcaloïdes de benzophénanthridine pour la préparation d'aliments ou d'additifs alimentaires pour l'amélioration du bilan protéinique chez des animaux nécessiteux par l'inhibition ou le blocage de la décomposition enzymatique des acides aminés aromatiques dans l'appareil digestif.

7. Utilisation d'alcaloïdes de benzophénanthridine ou de leurs dérivés ou de leurs analogues synthétiques ou de composants végétaux des papaveraceae contenant un ou des alcaloïdes de benzophénanthridine pour la préparation d'un médicament pour le traitement des inflammations intestinales,
qui sont générées par les produits de décomposition des acides aminés tels que l'indole et le scatole.
